# EUROPEAN PATENT SPECIFICATION

(11) **EP 2 198 921 B1**
(45) Date of publication and mention of the grant of the patent: **11.09.2013**
(21) Application number: 09015584.7
(22) Date of filing: 16.12.2009
(51) Int. Cl.: A61N 5/06

(54) **Light-irradiating beauty care device**
Schönheitspflegevorrichtung mit Lichtabstrahlung
Dispositif de soins de beauté à éclairage lumineux

(30) Priority: 22.12.2008 JP 2008326528
(43) Date of publication of application: 23.06.2010
(73) Proprietor: Panasonic Corporation, Osaka 571-8501 (JP)
(72) Inventor: Sueyoshi, Hidekazu, Kadoma-shi Osaka (JP); Wang, Wei, Kadoma-shi Osaka (JP); Ogawa, Hitoshi, Kadoma-shi Osaka (JP)
(74) Representative: Samson & Partner

(56) References cited:
- WO-A1-2008/088792
- WO-A2-2008/052152
- US-A1- 2007 179 574
- US-A1- 2007 219 600
- US-A1- 2008 215 123

## Description

### Field of the Invention

The present invention relates to a light-irradiating beauty care device and, more specifically, to a light-irradiating beauty care device provided with a light source and designed to treat a skin by irradiating the light generated from the light source on a living body surface, particularly on a skin surface.

### Background of the Invention

Conventionally, there has been available a light-irradiating beauty care device that treats a skin to remove hair or to suppress revival of hair by irradiating the light generated from a light source on a living body surface, particularly on a skin surface.

It is, however, sometimes the case that the beauty care device causes damage to the living body. In some cases, the light leaked out during irradiation gets into the eyes. In other cases, the living body surface makes direct contact with a light irradiation unit and gets burnt by the heat of the light irradiation unit.

In view of this, Japanese Patent Laid-open Publication No. 2004-321401 discloses a light-irradiating beauty care device in which a spacer is arranged between a light irradiation unit and a skin surface. The spacer prevents the skin surface from making contact with the light irradiation unit and assists in positioning the light irradiation unit with respect to the skin surface.

With this beauty care device, however, the light uniformly irradiated from the light irradiation unit becomes non-uniform in the spacer. More specifically, due to the polarization of the light in the spacer, the light is changed in its intensity and direction and is leaked from the side surfaces of the spacer. In addition, the light is leaked out through a gap formed between the spacer and the skin surface. This means that the safety of the beauty care device is not so high. Furthermore, the quantity of the light irradiated on the skin may sometimes be reduced as a result of the light becoming non-uniform. If the light source is upgraded in order to increase the quantity of the light, a great amount of heat is generated when emitting the light. This necessitates use of a heat radiator unit with an increased capacity, which results in an increase in device size and makes it difficult to use the beauty care device.

Japanese Patent Laid-open Publication No. 2005-278724 discloses a light-irradiating beauty care device in which a touch sensor mechanism is provided in a light irradiation unit. In this beauty care device, a plurality of sensors is used to detect whether the device faces toward, and makes contact with, a skin surface. This ensures that a gap is hardly formed between the light irradiation unit and the skin surface, thereby suppressing leakage of the light and increasing the safety.

However, use of the touch sensor mechanism results in an increase in device weight and size. Since the resistance value of the skin surface varies depending on the conditions thereof, the sensors may possibly suffer from inaccurate operation. For these reasons, the beauty care device is not so convenient to use.

WO 2008/088792 A1 discloses a light-irradiating beauty care device according to the preamble of claim 1.

### Summary of the Invention

While the invention is defined in the independent claim 1, further aspects of the invention are set forth in the dependent claims, the drawings and the following description.

In view of the above, the present invention provides an easy-to-use, safe, light-irradiating beauty care device capable of preventing reduction of the quantity of light irradiated on a living body surface without increasing the size of the device, capable of preventing the living body surface from making contact with a light irradiation unit, and capable of preventing leakage of the irradiated light by making the light irradiation unit directed toward the living body surface in a reliable manner.

In accordance with an aspect of the present invention, there is provided a light-irradiating beauty care device including: a hand-held main body and a light irradiation unit provided at one end of the main body for irradiating light on a living body surface, the light irradiation unit including a light source for emitting the light and a lens through which to pass the light. The light-irradiating beauty care device further includes a light distribution control unit having a discharge portion provided between the lens of the light irradiation unit and the living body surface and an opaque portion for covering the peripheries of the discharge portion and the light irradiation unit. The discharge portion includes optical members through which to transmit the light irradiated from the light irradiation unit while maintaining the quantity of light.

With this configuration, it is possible for the opaque portion to prevent the light irradiated by the light irradiation unit from being leaked to other portions than the discharge portion. Furthermore, the light can be irradiated on the living body surface while keeping the living body surface out of contact with the light irradiation unit.

The light-irradiating beauty care device may further include irradiation trigger switches for, when operated, allowing the light irradiation unit to irradiate the light, and the light distribution control unit may be movable along a light irradiation direction perpendicular to the living body surface relative to the lens, and at least one of the irradiation trigger switches may be operable in response to the movement of the light distribution control unit along the light irradiation direction.

In this case, it is possible to assure close contact between the light distribution control unit and the living body surface. One of the irradiation trigger switches is operated by pressing the light distribution control unit against the living body surface. This makes it possible to reliably irradiate the light on the living body surface and to enhance the ease of use of the beauty care device.

The light distribution control unit may be held in the main body to be movable along the light irradiation direction. Thus, the light distribution control unit is smoothly moved in the light irradiation direction F. This assures close contact and enhanced safety, while improving the ease of use of the beauty care device.

The light-irradiating beauty care device may further include a lock unit for inhibiting the light distribution control unit from moving in the light irradiation direction. Provision of the lock unit eliminates the fear of inadvertent operation of the beauty care device otherwise caused by the falling shock of the beauty care device and the fear of erroneous light irradiation otherwise caused by the mischief of a child. This makes it possible to prevent the light from being irradiated on other regions than the living body surface to be subjected to beauty care, thereby assuring enhanced safety.

The light distribution control unit may include an attachment portion removably attached to the main body. This makes it possible to replace the light distribution control unit with ease in case of occurrence of deterioration or trouble, which further enhances the safety and the ease of use of the beauty care device.

The optical members of the discharge portion are preferably arranged in a grid shape.

Preferably, the discharge portion and the opaque portion of the light distribution control unit are integrally formed with each other.

Preferably, the light source of the light irradiation unit includes a xenon tube or a diode.

With the light-irradiating beauty care device of the present invention noted above, the entry of the living body surface into the beauty care device is restrained by the light distribution control unit, particularly the optical members arranged in the discharge portion. The positioning of the light irradiation unit and the light irradiation operation can be performed with no fear that the living body makes contact with the light irradiation unit. Leakage of the light is reliably prevented by the opaque portion. The discharge portion keeps the light irradiated from the light irradiation unit while maintaining the quantity of the light passing through the discharge portion. Accordingly, the light-irradiating beauty care device can safely and reliably perform beauty care to the living body surface by light irradiation without impairing the ease of use.

### Brief Description of the Drawings

Figs. 1A and 1B are front and side views showing a light-irradiating beauty care device in accordance with an embodiment of the present invention.
Figs. 2A and 2B are section views taken along line 2A-2A in Fig. 1A and line 2B-2B in Fig. 1B, respectively.
Fig. 3 is a perspective view of the beauty care device shown in Figs. 1A and 1B, with a cover removed for clarity.
Fig. 4A is a front view illustrating a light irradiation unit employed in the beauty care device and Fig. 4B is a section view taken along line 4B-4B in Fig. 4A.
Fig. 5 is an exploded perspective view showing the light irradiation unit employed in the beauty care device.
Fig. 6 is a perspective view showing a light distribution control unit employed in the beauty care device.
Fig. 7A is a front view showing a cover employed in the beauty care device and Fig. 7B is a section view taken along line 7B-7B in Fig. 7A.
Fig. 8A is a front view of the cover with the light distribution control unit remaining in a slide-prevented state and Fig. 8B is a section view taken along line 8B-8B in Fig. 8A.

### Detailed Description of the Preferred Embodiments

Hereinafter, one embodiment of the present invention will be described with reference to the accompanying drawings.

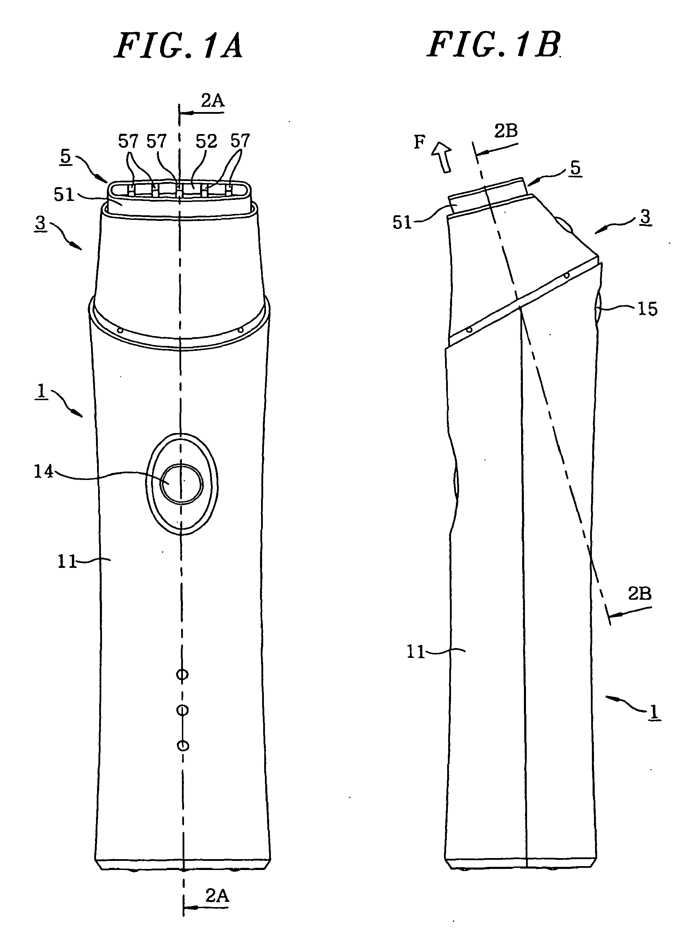

A light-irradiating beauty care device 1 in accordance with an embodiment of the present invention is configured to irradiate light on a living body surface, particularly on a skin surface, to perform beauty care such as light-aided hair removal by which to remove hair from the living body surface or light-aided hair growth suppression by which to suppress revival and growth of hair on the living body surface. Referring to Figs. 1A through 3, the beauty care device 1 includes a main body 11 which can be held with one hand, a light irradiation unit 4 removably attached to one end of the main body 11 and provided with a light source 41 therein, a light distribution control unit 5 for covering the light irradiation unit 4 and controlling the distribution of the irradiated light, and a cover 3 removably attached to the main body 11 for holding the light distribution control unit 5 in place.

A control circuit 13 for controlling the irradiation of light and a built-in power source 12 are provided inside the main body 11. A power switch 14 for turning on or off the power source 12 and a removal operation unit 15 used in removing the cover 3 are provided to the outer surface of the main body 11. Preferably, the control circuit 13 performs, e.g., light quantity control during long-term use by storing the number of continuous light irradiation and the number of total light irradiation of the light irradiation unit 4 and controlling the light-emitting quantity of the light source 41 based on the number of light irradiation thus stored. It is also preferable for the control circuit 13 to perform, e.g., safety management by detecting the temperature of the light irradiation unit 4 or the presence and absence of the cover 3 and the light irradiation unit 4. If necessary, the control circuit 13 may be modified to other designs.

At the light-irradiating end portion 2 of the main body 11 to which the light irradiation unit 4 is attached, there are provided unit holder portions 21 for removably holding the light irradiation unit 4, a body side locking portion 23 operable by the removal operation unit 15 and capable of releasably locking the cover 3 and a light-emitting switch 22 for causing the light source 41 of the light irradiation unit 4 to emit light.

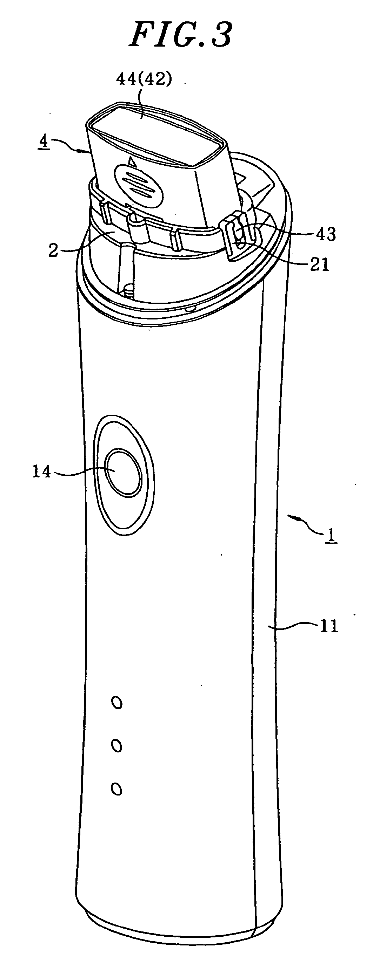

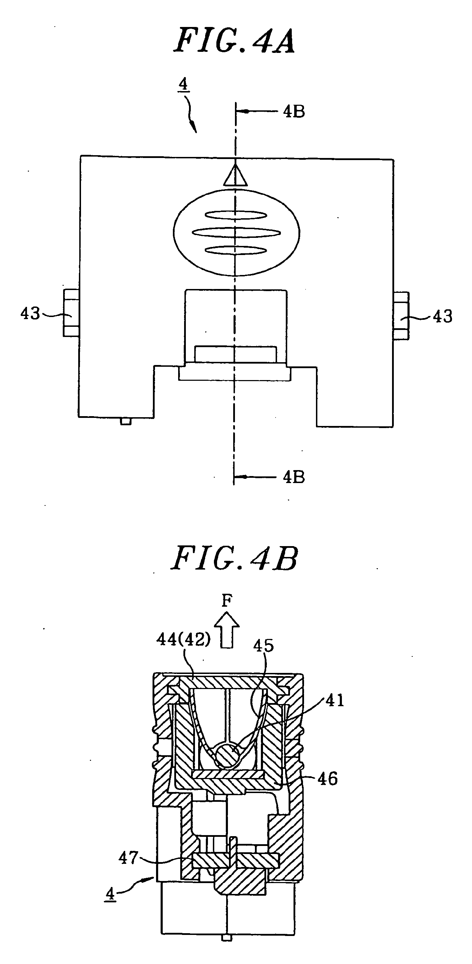

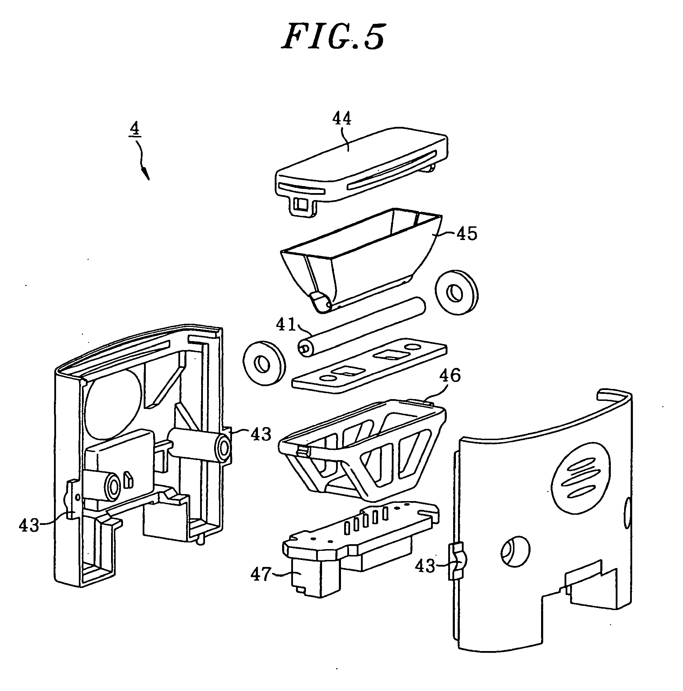

As shown in Figs. 3 through 5, the light irradiation unit 4 includes a box-shaped casing having at one end a generally rectangular irradiation mouth 42 through which to irradiate the light. A lens 44 as a lid is fitted to the irradiation mouth 42. Installation lugs 43 engageable with the unit holder portions 21 are formed at the lateral opposite end surfaces of the box-shaped casing, i.e., at the opposite end surfaces of the box-shaped casing in the longitudinal direction of the irradiation mouth 42. The light irradiation unit 4 is removably attached to the main body 11 by the engagement of the installation lugs 43 with the unit holder portions 21.

Within the box covered with the lens 44, there are arranged a xenon tube of tubular bulb shape serving as the light source 41, a reflector 45 for reflecting the light emitted from the light source 41 toward the irradiation mouth 42, a base 46 for holding the reflector 45 within the box and a unit circuit 47 for causing the light source 41 to emit light in response to a signal supplied from the light-emitting switch 22. The reflector 45 has a cup shape having a generally U-shape section, and includes an opening formed near the irradiation mouth 42 and a reflecting surface arranged at the inner side thereof. The size of the opening is substantially the same as that of the irradiation mouth 42.

The light source 41 is positioned on the bottom portion of the cup-shaped reflector 45 in the opposite position from the opening. The light emitted from the light source 41 is made uniform by the reflector 45 and the lens 44. More specifically, the direction of the light emitted from the light source 41 is made perpendicular to the irradiation mouth 42, and the distribution of the light irradiated from the irradiation mouth 42 is made substantially uniform. The light source 41 of the light irradiation unit 4 may be a single diode or a plurality of diodes.

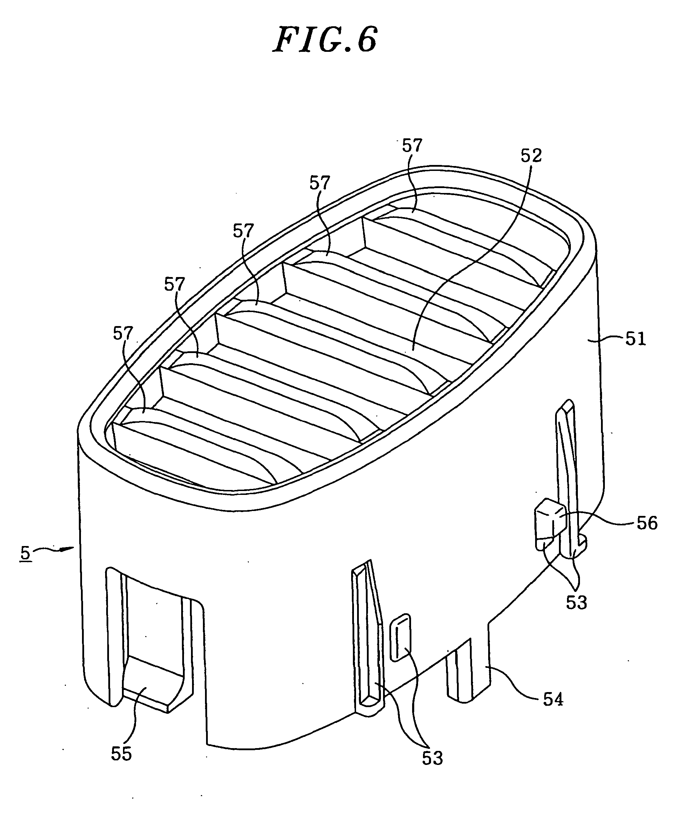

Referring to Fig. 6, the light distribution control unit 5 includes a light interrupting portion 51 of tubular shape made of an opaque material through which no light passes and a discharge portion 52 with optical members 57 provided at one generally-rectangular end opening of the light interrupting portion 51.

The light interrupting portion 51 has a tubular shape and includes a wall surface covering the side surfaces of the box-shaped casing of the light irradiation unit 4 and extending along the light irradiation direction F in which the light irradiation unit 4 irradiates light from the irradiation mouth 42. The opening of the light interrupting portion 51 is arranged corresponding to the irradiation mouth 42 of the light irradiation unit 4.

The opening of the light interrupting portion 51 serves as a discharge mouth through which the light from the light irradiation unit 4 is discharged to the outside. The end portion of the light interrupting portion 51 defining the discharge mouth makes contact with the living body surface during irradiation of the light. The light irradiation direction F refers to the direction perpendicular to the plane of the irradiation mouth 42, namely the direction in which the light made uniform by the reflector 45 and the lens 44 is irradiated from the irradiation mouth 42.

Slide guides 53 extending along the light irradiation direction F perpendicular to the opening of the light interrupting portion 51 are formed on the opposite wider side surfaces of the light interrupting portion 51. When the parts of the light-irradiating beauty care device 1 are assembled together, the slide guides 53 allow the light distribution control unit 5 to slidingly move in a direction parallel to the light irradiation direction F.

At the generally center point of the end side of one of the opposite wider side surfaces of the light interrupting portion 51 that defines an opening opposite from the discharge mouth, there is provided a switch-operating protrusion 54 extending from the end side in a parallel relationship with the opposite wider side surfaces of the light interrupting portion 51. The switch-operating protrusion 54 operates the light-emitting switch 22 as the light distribution control unit 5 is slid toward the light-emitting switch 22 under the guidance of the slide guides 53.

More specifically, if the portion of the light distribution control unit 5 defining the discharge mouth is brought into contact with the living body surface and pressed against the living body surface in the light irradiation direction F or press-contacted with the living body surface with the power switch 14 turned on, the light distribution control unit 5 is slid in the opposite direction from the light irradiation direction F. Upon this sliding movement, the switch-operating protrusion 54 presses the light-emitting switch 22, as a result of which the light-emitting switch 22 energizes the light source 41 through the unit circuit 47. At the end of light irradiation, the light distribution control unit 5 is slid in the light irradiation direction F, whereby the switch-operating protrusion 54 is moved away from the light-emitting switch 22 to remove the pressure applied to the light-emitting switch 22.

The light irradiation operation mentioned above is nothing more than one example and may be appropriately modified. For example, the light irradiation may be performed by turning on the power switch 14 in a state that the light distribution control unit 5 is pressed against the living body surface to energize the light-emitting switch 22. Other irradiation switches may be employed insofar as they are good enough to attain the advantageous effects of the present invention.

Retainer hooks 55 facing outwards are formed on the opposite narrower side surfaces of the light interrupting portion 51 adjacent to the opposite wider side surfaces. The retainer hooks 55 are fitted to the cover 3 to have the light distribution control unit 5 held in the cover 3, thereby preventing the light distribution control unit 5 from being removed and getting loosened during its sliding movement.

The discharge portion 52 provided in the discharge mouth is a grid that includes the optical members 57 positioned not to protrude beyond the discharge mouth in the light irradiation direction F, namely positioned flush with or inwardly of the end surface of the light interrupting portion 51. The grid is capable of transmitting the light made uniform by the light irradiation unit 4 having the convex or concave lens while maintaining the quantity of light. The grid is integrally formed with the light interrupting portion 51.

When the light distribution control unit 5 is brought into contact with the living body surface, the discharge portion 52 restrains the living body surface from coming into the internal space of the light interrupting portion 51. This prevents the living body surface from making contact with the light irradiation unit 4, particularly the lens 44. The optical members 57 are made of a material that does not change the intensity and direction of the light coming from the light irradiation unit 4 nor make the quantity of light non-uniform. Otherwise, the optical members 57 are formed into a shape that does not make the quantity of light non-uniform.

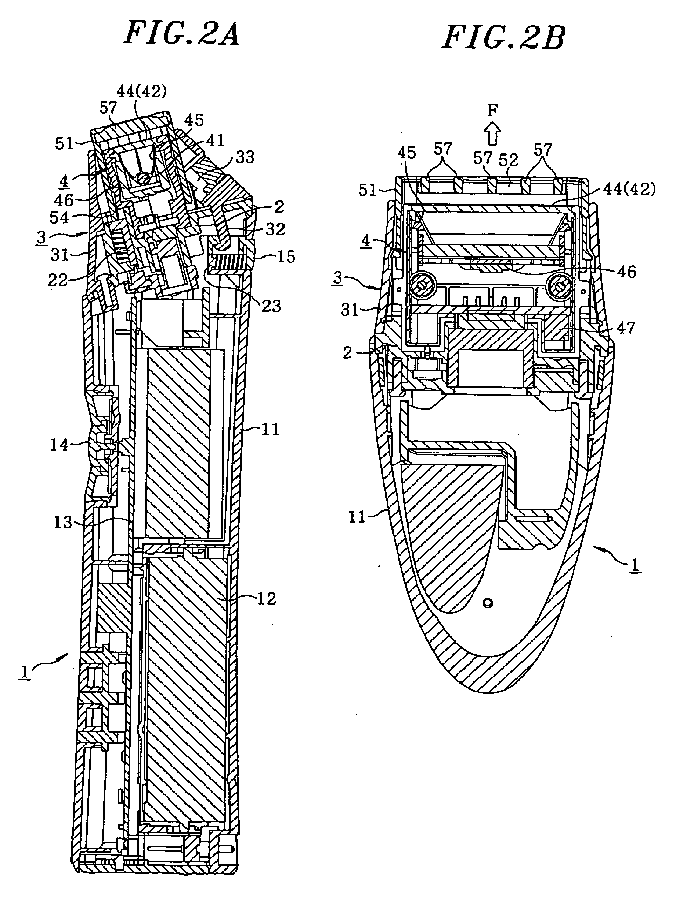

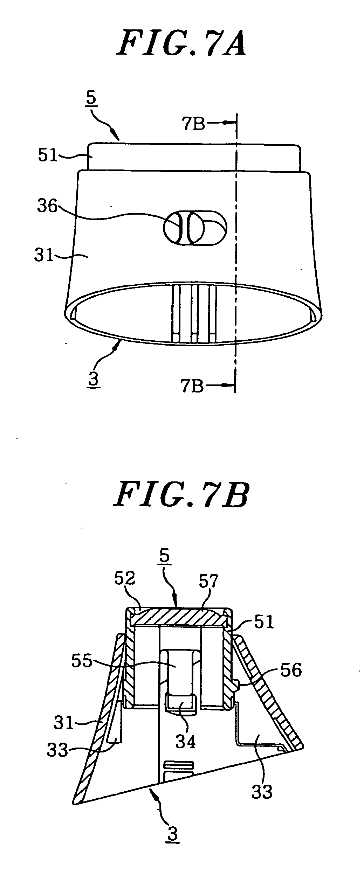

As shown in Figs. 2A, 2B, 7A and 7B, the cover 3 includes a cover member 31 provided with an opening for slidably holding the light distribution control unit 5 and designed to cover the light-irradiating end portion 2 of the main body 11 and the light irradiation unit 4.

The cover member 31 includes a locking hook 32 engageable with the body side locking portion 23, guide ribs 33 for supporting the slide guides 53 and retainer hook rests 34 for holding the retainer hooks 55 in place. The guide ribs 33 are formed inside the cover member 31 to extend along the light irradiation direction F. The guide ribs 33 are configured to support the light distribution control unit 5 so that, when the retainer hooks 55 and the retainer hook rests 34 are fitted together, the light distribution control unit 5 can make sliding movement while the slide guides 53 are guided by the slide ribs 33. Resilient bodies (not shown), e.g., springs, for biasing the retainer hooks 55 in the light irradiation direction F are provided in the retainer hook rests 34. The light distribution control unit 5 is biased in the light irradiation direction F by the resilient bodies and held in the cover 3 in such a position where the switch-operating protrusion 54 does not press the light-emitting switch 22.

As shown in Figs. 6 through 8B, there is provided a lock unit for inhibiting the sliding movement of the light distribution control unit 5. The lock unit includes a protruded lock portion 56 formed in the light distribution control unit 5 to protrude perpendicularly from the surface of the light distribution control unit 5 along which the slide guides 53 extend. The lock unit further includes a stopper 35 engageable with the protruded lock portion 56 and a lock operating portion 36 with which to operate the stopper 35 to engage with or disengage from the protruded lock portion 56. The stopper 35 and the lock operating portion 36 are provided in the cover 3.

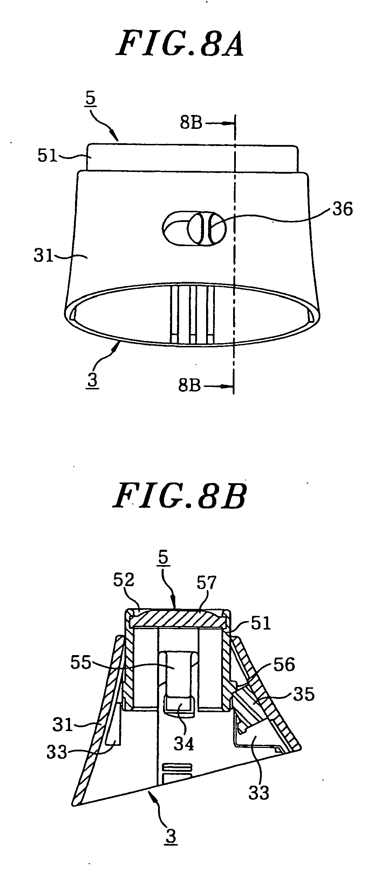

More specifically, the lock operating portion 36 and the stopper 35 are integrally formed with each other. If the lock operating portion 36 is shifted in the direction perpendicular to the sliding direction of the light distribution control unit 5 from the position shown in Fig. 7A in which the light distribution control unit 5 is free to slide, the stopper 35 is also moved in the direction perpendicular to the sliding direction of the light distribution control unit 5. Thus, the stopper 35 comes into engagement with the protruded lock portion 56 as shown in Fig. 8B. As a result, the light distribution control unit 5 is held in the cover 3 against any sliding movement. If the lock operating portion 36 is returned to the position shown in Fig. 7A, the stopper 35 disengages from the protruded lock portion 56, thereby permitting the sliding movement of the light distribution control unit 5.

Provision of the light distribution control unit 5 eliminates the need to bring the light irradiation unit 4 into direct contact with the living body surface. The light interrupting portion 51 covering the periphery of the light irradiation unit 4 prevents the light from being leaked from the surface parallel to the light irradiation direction F. The discharge portion 52 having the optical members 57 arranged in a grid shape restrains the living body from coming into the light-irradiating beauty care device 1. This can reliably eliminate the fear that the living body makes contact with the light irradiation unit 4 and suffers from a burn. The discharge portion 52 makes uniform the quantity of the light irradiated from the light irradiation unit 4 and does not cause any change in the irradiated light. Accordingly, the light-irradiating beauty care device 1 can stably perform beauty care by irradiating a uniform quantity of light on the living body surface.

As the light distribution control unit 5, more precisely the discharge portion 52 or the end of the light interrupting portion 51 defining the discharge mouth, is pressed against the living body surface, the light distribution control unit 5 makes sliding movement to operate the light-emitting switch 22 so that the light source 41 can emit light. Thus, the light irradiation can be performed in a state that the discharge mouth and the irradiation mouth 42 definitely face toward the living body surface. Two switches, i.e., the power switch 14 and the light-emitting switch 22, are used as irradiation trigger switches for triggering the light irradiation. The two irradiation trigger switches are independently operated to trigger the light irradiation, one when turning on the power source and the other when pressing the discharge portion 53 against the living body surface. This can eliminate the fear that the light is mistakenly irradiated on other regions than the living body surface. If the light distribution control unit 5 is pressed against the living body surface, no gap is left between the light distribution control unit 5 and the living body surface. This helps assure close contact between the light distribution control unit 5 and the living body surface, thereby reliably preventing leakage of the light through between the light distribution control unit 5 and the living body surface. Thanks to these features, the light-irradiating beauty care device is easy and safe to use.

The light irradiation unit 4 and the light distribution control unit 5 are susceptible to deterioration or trouble during long-term use due to their frequent contact with the living body surface and the heat generated in the light emitting process. Since the light irradiation unit 4 and the light distribution control unit 5 are attached to the main body 11 in an easily removable manner, they can be removed and replaced with new ones in case of occurrence of deterioration or trouble. In this case, since the light interrupting portion 51 and the discharge portion 52 of the light distribution control unit 5 are integrally formed with each other and the light irradiation unit 4 is formed into a single unit, there is no fear that the discharge portion 52 and the light source 41 are misaligned in the replacement process, which would otherwise render the quantity of light non-uniform. This makes it possible to replace defective parts with ease, which further enhances the safety and the ease of use of the light-irradiating beauty care device.

By having the light distribution control unit 5 held in the cover 3, the light distribution control unit 5 can make smooth sliding movement with no looseness when a user grips the main body 11 and presses the light distribution control unit 5 against the living body surface. This assures close contact between the light distribution control unit 5 and the living body surface and allows the switch-operating protrusion 54 to press the light-emitting switch 22 in a reliable manner. If the light distribution control unit 5 is moved away from the living body surface after light irradiation, the resilient body biases the switch-operating protrusion 54 into a position where the switch-operating protrusion 54 does not press the light-emitting switch 22. This eliminates the need to return the light distribution control unit 5 to an original position or to otherwise adjust the position thereof. This also prevents misalignment of the light distribution control unit 5 with respect to the light source 41. In addition, it is possible to prevent inadvertent operation of the light-emitting switch 22 while the beauty care device is not pressed against the living body surface.

Provision of the lock unit for inhibiting the sliding movement of the light distribution control unit 5 eliminates the fear of inadvertent operation of the beauty care device otherwise caused by the falling shock of the beauty care device and the fear of erroneous light irradiation otherwise caused by the mischief of a child. This makes it possible to prevent the light from being irradiated on other regions than the living body surface, thereby assuring enhanced safety. The engaging or coupling parts and the slide mechanisms of the light-irradiating beauty care device are not limited to the configuration described above but may be modified to other configurations insofar as the advantageous effects of the present invention are attainable.

While the invention has been shown and described with respect to the embodiments, it will be understood by those skilled in the art that various changes and modifications may be made without departing from the scope of the invention as defined in the following claims.

## Claims

1. A light-irradiating beauty care device (1) comprising:
a hand-held main body (11);
a light irradiation unit (4) provided at one end of the main body (11) for irradiating light on a living body surface, the light irradiation unit (4) including a light source (41) for emitting the light and a lens (44) through which to pass the light; and
a light distribution control unit (5) including a discharge portion (52) provided between the lens (44) of the light irradiation unit (4) and the living body surface and an opaque portion (51) for covering the peripheries of the discharge portion (52) and the light irradiation unit (4), the discharge portion (52) having optical members (57) through which the light irradiated from the light irradiation unit (4) is transmissible while maintaining the quantity of light, **characterized in that**
the light distribution control unit (5) is held in the main body (11) in a manner that the light distribution control unit (5) is movable relative to the lens (44) in a light irradiation direction (F) in which the light irradiation unit (4) irradiates the light, and
the light distribution control unit (5) further includes one or more slide guides (53) formed on side surfaces of the opaque portion (51), the slide guides (53) allowing the light distribution control unit (5) to slidingly move in the light irradiation direction (F) and in a direction opposing thereto.

2. The device (1) of claim 1, further comprising irradiation trigger switches (14, 22) for allowing the light irradiation unit (44) to irradiate the light, at least one of the irradiation trigger switches (14, 22) being operable in response to the movement of the light distribution control unit (5) along the light irradiation direction (F).

3. The device (1) of claim 1 or 2 further comprising a lock unit (56) for inhibiting the light distribution control unit (5) from moving in the light irradiation direction (F).

4. The device (1) of any one of claims 1 to 3, wherein the light distribution control unit (5) includes an attachment portion (55) removably attached to the main body.

5. The device (1) of any one of claims 1 to 4, wherein the optical members (57) of the discharge portion (52) are arranged in a grid shape.

6. The device (1) of any one of claims 1 to 5, wherein the discharge portion (52) and the opaque portion (51) of the light distribution control unit (5) are integrally formed with each other.

7. The device (1) of any one of claims 1 to 6, wherein the light source (41) of the light irradiation unit (4) comprises a xenon tube or a diode.

8. The device (1) of any one of claims 1 to 7, wherein the opaque portion (51) has an end portion forming an opening at which the optical members (57) are provided, and
wherein the optical members (57) are positioned flush with or inwardly of the end portion of the opaque portion (51).

## Patentansprüche

1. Lichtabstrahlschönheitspflegevorrichtung (1) umfassend:
einen tragbaren Hauptkörper (11);
eine Lichtabstrahleinheit (4), die an einem Ende des Hauptkörpers (11) für ein Abstrahlen von Licht auf eine lebende Körperfläche vorgesehen ist, wobei die Lichtabstrahleinheit (4) eine Lichtquelle (41) zum Emittieren des Lichts und eine Linse (44) umfasst, durch die das Licht passiert; und
eine Lichtverteilungssteuereinheit (5) mit einem Abgabebereich (52), der zwischen der Linse (44) der Lichtabstrahleinheit (4) und der lebenden Körperfläche vorgesehen ist und einen lichtundurchlässigen Bereich (41) zum Abdecken der Umfänge des Abgabebereichs (52) und der Lichtabstrahleinheit (4), wobei der Abgabebereich (52) optische Elemente (57) umfasst, die für das Licht, das von der Lichtabstrahleinheit (4) abgestrahlt wird, durchlässig sind, wobei die Lichtquantität aufrechterhalten bleibt, **dadurch gekennzeichnet, dass**
die Lichtverteilungssteuereinheit (5) in dem Hauptkörper (11) derart gehalten ist, dass die Lichtverteilungssteuereinheit (5) relativ zu der Linse (44) in einer Lichtabstrahlrichtung (F) bewegbar ist, in der die Lichtabstrahleinheit (4) das Licht abstrahlt, und
die Lichtverteilungssteuereinheit (5) weiter umfasst eine oder mehrere Gleitführungen (53), die an Seitenflächen des lichtundurchlässigen Bereichs (51) ausgebildet sind, wobei die Gleitführungen (53) es der Lichtverteilungssteuereinheit (5) ermöglichen, sich in die Lichtabstrahlrichtung (F) und in eine Richtung entgegengesetzt dazu verschiebbar zu bewegen.

2. Vorrichtung gemäß Anspruch 1, weiter umfassend Abstrahlauslöseschalter (14, 22), um es der Lichtabstrahleinheit (44) zu ermöglichen, das Licht abzustrahlen, wobei wenigstens einer der Abstrahlauslöseschalter (14, 22) in Reaktion auf die Bewegung der Lichtverteilungssteuereinheit (5) entlang der Lichtabstrahlrichtung (F) betreibbar ist.

3. Vorrichtung (1) gemäß Anspruch 1 oder 2, weiter umfassend eine Sperreinheit (56), um die Lichtverteilungssteuereinheit (5) von einem Bewegen in die Lichtabstrahlrichtung (F) abzuhalten.

4. Vorrichtung (1) gemäß einem der Ansprüche 1 bis 3, bei der die Lichtverteilsteuereinheit (5) einen Befestigungsbereich (55) umfasst, der entfernbar an dem Hauptkörper befestigt ist.

5. Vorrichtung (1) gemäß einem der Ansprüche 1 bis 4, bei der die optischen Elemente (57) des Abgabebereichs (52) in einer Gitterform angeordnet sind.

6. Vorrichtung gemäß einem der Ansprüche 1 bis 5, bei der der Abgabebereich (52) und der lichtundurchlässige Bereich (51) der Lichtverteilungssteuereinheit (5) integral miteinander ausgebildet sind.

7. Vorrichtung gemäß einem der Ansprüche 1 bis 6, bei der die Lichtquelle (41) der Lichtabstrahleinheit (4) ein Xenonrohr oder eine Diode umfasst.

8. Vorrichtung gemäß einem der Ansprüche 1 bis 7, bei der der lichtundurchlässige Bereich (51) einen Endbereich aufweist, der eine Öffnung bildet, an der die optischen Elemente (57) vorgesehen sind, und
wobei die optischen Elemente (57) fluchtend mit oder nach innen gerichtet von dem Endbereich des lichtundurchlässigen Bereichs (51) positioniert sind.

## Revendications

1. Dispositif (1) de soins de beauté à projection de lumière comprenant :
un corps principal (11) portatif ;
une unité de projection de lumière (4) située au niveau d'une première extrémité du corps principal (11) destinée à projeter de la lumière sur une surface de corps vivant, l'unité de projection de lumière (4) comprenant une source de lumière (41) destinée à émettre la lumière et une lentille (44) à travers laquelle passe la lumière ; et
une unité de commande (5) de répartition de lumière comprenant une partie de décharge (52) située entre la lentille (44) de l'unité de projection de lumière (4) et la surface de corps vivant et une partie opaque (51) destinée à recouvrir les périphéries de la partie de décharge (52) et de l'unité de projection de lumière (4), la partie de décharge (52) possédant des éléments optiques (57) à travers lesquels la lumière projetée par l'unité de projection de lumière (4) est transmissible tout en maintenant la quantité de lumière, **caractérisé en ce que**
l'unité de commande (5) de répartition de lumière est contenue dans le corps principal (11) d'une manière telle que l'unité de commande (5) de répartition de lumière soit mobile par rapport à la lentille (44) dans une direction de projection de lumière (F) dans laquelle l'unité de projection de lumière (4) projette la lumière, et
l'unité de commande (5) de répartition de lumière comprend en outre un ou plusieurs guides de coulissement (53) formés sur les surfaces latérales de la partie opaque (51), les guides de coulissement (53) permettant à l'unité de commande (5) de répartition de lumière de se déplacer de manière coulissante dans la direction de projection de lumière (F) et dans une direction opposée à celle-ci.

2. Dispositif (1) selon la revendication 1, comprenant en outre des commutateurs de déclenchement (14, 22) de projection destinés à permettre à l'unité de projection de lumière (44) de projeter la lumière, au moins un des commutateurs de déclenchement (14, 22) de projection pouvant fonctionner en réponse au déplacement de l'unité de commande (5) de répartition de lumière le long de la direction de propagation de lumière (F).

3. Dispositif (1) selon la revendication 1 ou 2 comprenant en outre une unité de verrouillage (56) destinée à empêcher l'unité de commande (5) de répartition de lumière de se déplacer dans la direction de propagation de lumière (F).

4. Dispositif (1) selon l'une quelconque des revendications 1 à 3, dans lequel l'unité de commande (5) de répartition de lumière comprend une partie de fixation (55) fixée amovible au corps principal.

5. Dispositif (1) selon l'une quelconque des revendications 1 à 4, dans lequel les éléments optiques (57) de la partie de décharge (52) sont agencés sous forme de grille.

6. Dispositif (1) selon l'une quelconque des revendications 1 à 5, dans lequel la partie de décharge (52) et la partie opaque (51) de l'unité de commande (5) de répartition de lumière ne forment qu'un seul bloc.

7. Dispositif (1) selon l'une quelconque des revendications 1 à 6, dans lequel la source de lumière (41) de l'unité de propagation de lumière (4) comprend un tube au xénon ou une diode.

8. Dispositif (1) selon l'une quelconque des revendications 1 à 7, dans lequel la partie opaque (51) possède une partie d'extrémité formant une ouverture au niveau de laquelle se trouvent les éléments optiques (57), et
dans lequel les éléments optiques (57) sont positionnés dans l'alignement ou vers l'intérieur de la partie d'extrémité de la partie opaque (51).
